# EUROPÄISCHE PATENTSCHRIFT

(11) **EP 2 805 735 B1**
(45) Veröffentlichungstag und Bekanntmachung des Hinweises auf die Patenterteilung: **04.07.2018**
(21) Anmeldenummer: 13168483.9
(22) Anmeldetag: 21.05.2013
(51) Int. Cl.: A61M 5/32, A61M 5/50, A61M 5/20, A61M 5/30, F41B 9/00

(54) **Injektionssystem zur Injektion eines großen Volumens**
Injection system for injecting a large volume
Système d'injection d'un grand volume

(43) Veröffentlichungstag der Anmeldung: 26.11.2014
(73) Patentinhaber: LTS LOHMANN Therapie-Systeme AG, 56626 Andernach (DE)
(72) Erfinder: Hoffmann, Hans-Rainer, 56566 Neuwied (DE); Horstmann, Michael, 56564 Neuwied (DE); Hadaschik, Roman, 99817 Eisenach (DE); Lell, Peter, 85368 Moosburg (DE)

(56) Entgegenhaltungen:
- EP-A1- 1 570 875
- EP-A1- 1 752 174
- WO-A2-2004/093818

## Beschreibung

Die Erfindung betrifft ein Injektionssystem mit einer mittels einer Auslösevorrichtung auslösbaren Antriebsvorrichtung und mit einem, mindestens eine Austrittsöffnung aufweisenden Medikamentenbehälter mit komprimierbarem Volumen.

Aus der DE 100 29 325 A1 ist eine derartige Vorrichtung bekannt. Bei einer Injektion eines großen Medikamentenvolumens können Hautausbeulungen, sogenannte Quaddeln auftreten. Aufgrund des in den Quaddeln entstehenden hohen Drucks besteht die Gefahr eines ungesteuerten Rückflusses von Medikamentenlösung. Dies kann zu einer Unterdosierung des Medikamentes im Körper führen.

Der vorliegenden Erfindung liegt die Problemstellung zugrunde, die genaue Dosierung eines großen Medikamentenlösungsvolumens zu ermöglichen.

Diese Problemstellung wird mit den Merkmalen des Hauptanspruchs gelöst. Dazu weist das Injektionssystem mindestens einen weiteren komprimierbaren Medikamentenbehälter auf. Alle Antriebsvorrichtungen sind mittels der gemeinsamen Auslösevorrichtung betätigbar.

Weitere Einzelheiten der Erfindung ergeben sich aus den Unteransprüchen und den nachfolgenden Beschreibungen schematisch dargestellter Ausführungsbeispiele.
- Figur 1:: Injektionssystem;
- Figur 2:: Schnitt durch eine Injektionseinheit;
- Figur 3:: Detail von Figur 1;
- Figur 4:: Detail eines Injektionssystems mit federnd gelagerten Injektionseinheiten;
- Figur 5:: Gitterartig angeordnetes Injektionssystem;
- Figur 6:: Injektionssystem mit mehreren, in einem gemeinsamen Gehäuse angeordneten Injektionseinheiten;
- Figur 7:: Längsschnitt durch ein Injektionssystem nach Figur 6;
- Figur 8:: Dimetrische Ansicht eines Injektionssystems nach Figur 6;
- Figur 9:: Schnitt durch eine Injektionseinheit mit Kolben.

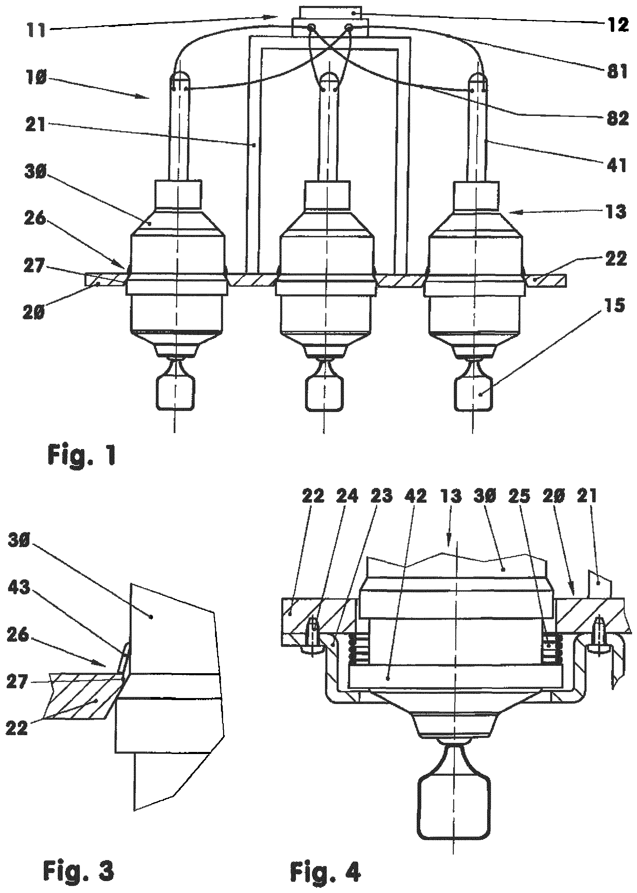

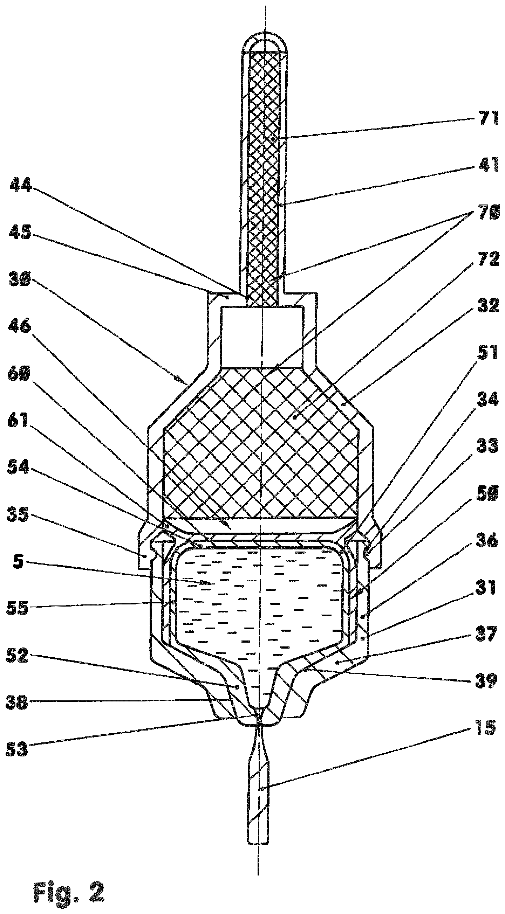

Die Figuren 1 und 2 zeigen ein Injektionssystem (10) in einer Ansicht und in einer Schnittdarstellung. Ein derartiges Einmal-Injektionssystem (10) wird beispielsweise eingesetzt, um ein großes Volumen eines Medikaments in einen Körper einzubringen. Das Injektionssystem (10) umfasst mehrere, in Injektionseinheiten (13) angeordnete Medikamentenbehälter (50), in denen jeweils ein Teilvolumen des gesamten Medikamentlösungsvolumens gelagert ist. Hierbei kann das im einzelnen Medikamentenbehälter (50) gelagerte Medikamentenvolumen gleich groß oder unterschiedlich groß sein. Auch kann die Art des Medikaments gleich oder unterschiedlich sein. Die Auslösung aller Antriebsvorrichtungen (70) erfolgt über eine gemeinsame Auslösevorrichtung (11).

Das in der Figur 1 dargestellte Injektionssystem (10) umfasst einen Systemträger (20) mit einer Trägerplatte (22), auf der ein Abstützgestell (21) befestigt ist. Dieses Abstützgestell (21) trägt im Ausführungsbeispiel die Auslösevorrichtung (11). Die Auslösevorrichtung (11) umfasst beispielsweise eine hier nicht dargestellte Stromquelle und einen gegebenenfalls verriegelbaren Taster (12). Auch der Einsatz eines Schlagbügels oder Schlagdrahtes ist denkbar. Eine derartige Auslösevorrichtung (11) arbeitet ohne den Einsatz einer Stromquelle. Die Zündladung (71) wird - nach Lösen einer Arretierungsvorrichtung - durch kräftiges seitliches Aufschlagen auf das Zundröhrchen (41) gezündet.

Der Systemträger (20) trägt weiterhin drei Gehäuse (30), in denen jeweils ein Medikamentenbehälter (50) aufgenommen ist. Das einzelne Gehäuse (30) umfasst einen ersten, hier unten dargestellten Gehäuseteil (31) und einen zweiten, hier oben dargestellten zweiten Gehäuseteil (32). Das erste (31) und das zweite Gehäusteil (32) sind beispielsweise miteinander verrastet. Hierbei übergreift das zweite Gehäuseteil (32) das erste Gehäuseteil (31). Ein Ringnocken (33) des oberen Gehäusteils greift formschlüssig in eine Ringnut (34) des unteren Gehäuseteils (31) ein.

Das erste Gehäuseteil (31) ist trichterförmig ausgebildet. Es hat einen zylindrischen Abschnitt (36) und einen konischen Abschnitt (37). Der konische Abschnitt (37) umgreift einen zentralen Durchbruch (38). Die Wandstärke des konischen Bereichs (37) ist im Ausführungsbeispiel um 80 % dicker als die Wandstärke des zylindrischen Abschnitts (36). Der Durchmesser des zentralen Durchbruchs (38) entspricht in der Darstellung der Figur 2 dem Doppelten der Wandstärke des konischen Abschnitts (37).

Das glockenförmig ausgebildete zweite Gehäusteil (32) weist einen nach innen überstehenden Haltering (35) auf, der den oberen Rand des ersten Gehäuseteils (31) überdeckt. Das zweite Gehäuseteil (32) hat eine weitgehend konstante Wandstärke.

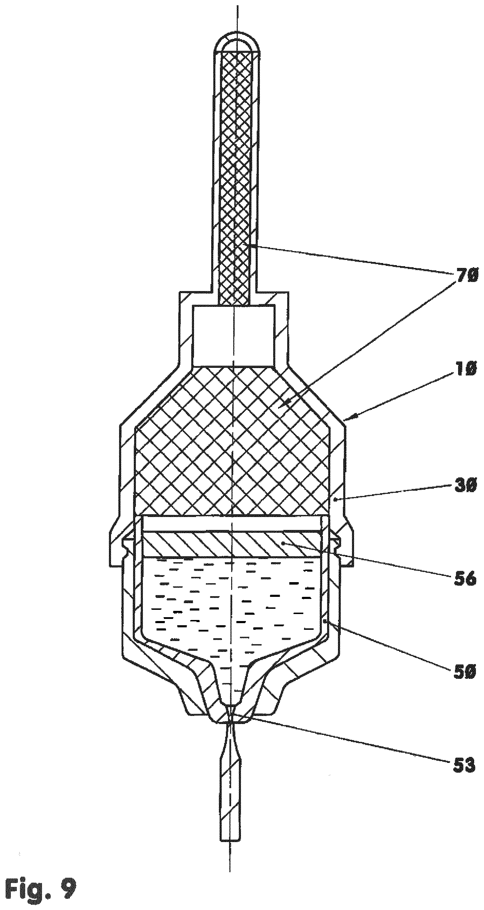

Der einzelne Medikamentenbehälter (50) hat im Ausführungsbeispiel eine kompressible Wandung (51). Es ist auch denkbar, einen inkompressiblen Behälter (50) einzusetzen, der beispielsweise einen verschiebbaren Kolben (56) umfasst, vgl. Figur 9.

Der Medikamentenbehälter (50) liegt an der Innenwandung (39) des konischen Abschnitts (37) an. Ein zentraler Austrittsabschnitt (52) mit einer Austrittsöffnung (53) ragt in der Darstellung der Figur 2 nach unten aus dem Gehäuse (30) heraus. Im Ausführungsbeispiel ist die Austrittsöffnung (53) als Düse (53) ausgebildet. Ihre Austrittsquerschnittsfläche beträgt beispielsweise 0,3 Promille der Innenquerschnittsfläche des Medikamentenbehälters (50). Die Austrittsöffnung (53) eines jeden Medikamentenbehälters (50) ist mittels eines Sterillverschlusses (15) verschlossen. Die Autrittsöffnung (53) kann auch ein Kanal einer Injektionsnadel oder einer Kanüle sein. Auch kann ein Medikamentenbehälter (50) mehrere Austrittsöffnungen (53) aufweisen. Der einzelne Medikamentenbehälter (50) ist z.B. mittels einer Medikamentenlösung (5) befüllt. Die Befüllung des Medikamentenbehälters (50) erfolgt beispielsweise in einem Blow-Fill-Verfahren, bei dem das Behältervolumen durch das Einpressen des flüssigen Medikaments (5) vergrößert wird. Das in allen Medikamentenbehältern (50) gelagerte gesamte Volumen des Medikaments (5) ist beispielsweise größer als 0,5 Milliliter.

Der Medikamentenbehälter (50) sitzt in einer topfförmig ausgebileten Schutzmenbran (60). Diese überdeckt die Oberseite (54) und die z.B. zylindrische Außenwandung (55) des Medikamentenbehälters (50). Die Schutzmembran (60) hat eine obenliegende Dichtlippe (61), die an der Innenwandung des zweiten Gehäuseteils (32) anliegt.

Die Antriebsvorrichtung (70) umfasst in diesem Ausführungsbeispiel pro Injektionseinheit (13) eine Zündladung (71) und eine Treibladung (72). Falls der mittels der Zündladung (71) erzeugte Gasdruck zum Auspressen der Medikamentenlösung (5) ausreicht, kann die Antriebsvorrichtung ohne Treibladung (72) ausgebildet sein.

Der Zünder kann beispielsweise beim Einsatz einer Gleichstromquelle als Batteriezünder ausgebildet sein. Aber auch der Einsatz eines Schlagzünders, Schlagbügels, Schlagdraht oder eines Piezozünders ist denkbar. Die Zündladung (71) ist in einem z.B. stabförmigen Gehäuseansatz (41), dem Zündröhrchen (41), des zweiten Gehäuseteils (32) angeordnet. In der Zündladung (71) ist beispielsweise eine Glühwendel oder ein Glühdraht angeordnet, der mittels zweier Leitungen (81, 82) mit der Auslösevorrichtung (11) verbunden ist. Eine der beiden Leitungen kann eine elektrische Masse sein. Sobald der Stromkreis mittels des Tasters (12) geschlossen ist, fließt Strom aus der Gleich- oder Wechselstromquelle über die Leitungen (81, 82), die Glühwendel und den Taster (12) and den jeweils anderen Pol der Stromquelle. In diesem Auslösestromkreis können ohmsche und/oder induktive und/oder kapazitive Bauteile angeordnet sein. Hiermit kann beispielsweise der Strom durch die einzelnen Glühwendeln und damit deren Erhitzung gesteuert werden. Die Auslösestromkreise für die einzelnen Zündladungen (71) können damit unterschiedlich sein, so dass die je eine Zündung bewirkende Erwärmung der einzelnen Zündladungen (71) des Injektorsystems (10) zu unterschiedlichen Zeitpunkten erfolgt. Es ist auch denkbar, z.B bei Einsatz eines Schiebeschalters anstatt des Tasters, einzelne Auslösestromkreise zeitlich nacheinander zu aktivieren. Auch eine getrennte Ansteuerung einzelner Zündladungen (71) ist möglich.

Anstelle der Glühwendel oder des Glühdrahtes ist auch eine Zündung der Zündladung (71) durch direkten Stromdurchgang durch die Zündladung (71) selbst möglich. Die Zündladung (71) kann zur Erhöhung der Leitfähigkeit z.B. Kohle- oder Grafitzusätze enthalten.

Die expandierende Zündladung (71) wirkt auf die im zweiten Gehäuseteil (32) angeordnete Treibladung (72). Diese Treibladung (72) füllt in der Darstellung der Figur 2 beispielsweise einen großen Teil des Innenraums (46) des zweiten Gehäuseteils (32) oberhalb der Schutzmembran (60) aus.

Sobald die Treibladung (72) mittels der Zündladung (71) aktiviert ist, erzeugt sie Gas, das auf die Schutzmembran (60) drückt. Gegebenenfalls kann die Schutzmembran (60) hierbei zerstört werden. Der Medikamentenbehälter (50) wird komprimiert und die Medikamentenlösung (5) wird - bei abgenommenem Sterilverschluss (15) - durch die Düse (53) ausgepresst. Die Außenwandung (55) und die Oberseite (54) des Medikamentenbehälters (50) werden verformt. Hierbei verhindern die verstärkten Bereiche des unteren Gehäuseteils (31) eine Beschädigung und/oder eine Zerstörung des Gehäuses (30). Bei einem starren Medikamentenbehälter (50) wird der Kolben (56) in Richtung der Austrittsöffnung (53) verschoben und damit das Volumen im Medikamentenbehälter (50) verringert.

Die einzelnen Gehäuse (30) sind in der Darstellung der Figur 1 beispielsweise mittels einer Rastverbindung (26) in der Trägerplatte (22) des Systemträgers (20) befestigt. In der Figur 3 ist dies vergrößert dargestellt. Hierzu verfügt das Gehäuse (30) über elastisch verformbare Raststifte (43), die bei der Montage die Trägerplatte (22) hintergreifen. Der Aufnahmedurchbruch (27) ist im Ausführungsbeispiel konisch ausgebildet, so dass jedes einzelne Gehäuse (30) fest im Systemträger (20) sitzt.

Zum Einsatz des Injektorsystems (10) wird dieses auf die Haut aufgesetzt und gegebenenfalls z.B. mittels Bändern gesichert. Nach dem Betätigen der Auslösevorrichtung (11) zünden beispielsweise alle angeschlossenen Zündladungen (71), die wiederum die gegebenenfalls vorhandenen Treibladungen (72) antreiben. Das Medikament wird aus allen Medikamentenbehältern (50) mit hohem Druck ausgepresst und penetriert die Haut. Bei der Kompression werden der Medikamentenbehälter (50) und die Schutzmembran (60) in radialer Richtung mittels der Innenwandung (39) geführt. Die Injektion des gesamten Medikamentenvolumens erfolgt in einem Zeitintervall, das kleiner ist als eine Sekunde. Beispielsweise liegt das Zeitintervall zwischen null und 200 Millisekunden.

Aufgrund der Vielzahl der Austrittsdüsen (53) ist das Medikamentenvolumen nach dem Einbringen unter der Hautoberfläche verteilt. Hiermit werden Hautausbeulungen durch eine Medikamentenanhäufung vermieden. Auch ein ungesteuerter Rückfluß der Medikamentenlösung, der zu einer Unterdosierung des Medikaments im zu behandelnden Körper führen kann, wird verhindert.

Die Figur 4 zeigt eine weitere Ausführungsform des Injektionssystems (10). Hier sind die einzelnen Gehäuse (30) federnd in der Trägerplatte (22) gelagert. Ein das erste Gehäuseteil (31) umgebender Ringbund (42) liegt unterhalb einer Trägerplatte (22) und ist an dieser mittels Federelementen (25), z.B. Druckfedern, abgestützt. Anstatt des jeweils ein Gehäuse (30) umgebenden Federelements (25) können auch andere Federelemente oder elastisch verfombare Körper eingesetzt werden.

Der Ringbund (42) ist im Ausführungsbeispiel der Figur 3 von einer Abhebesicherung (23) untergriffen. Dies ist beispielsweise ein Blech, das mittels Schrauben (24) an der Trägerplatte (22) befestigt ist.

Diese Ausführungsform ermöglicht ein gleichmäßigeren Eindruck der einzelnen Einheiten (13) in die Haut. Das Injektionssystem (10) passt sich an die Hautoberfläche an. Damit ist eine erhöhte Sicherheit für eine gleichförmige und vollständige Übertragung des Medikaments (5) aus allen Medikamentenbehältern (50) durch die Austrittsdüsen (53) hindurch und unter die Haut gegegben. Die Gefahr eines sogenannten "wet shots", bei dem aufgrund eines zu großen Abstands zwischen der einzelnen Austrittsdüse (53) und der Hautoberfläche Medikamentenlösung seitlich austreten kann, ist damit weiter verringert.

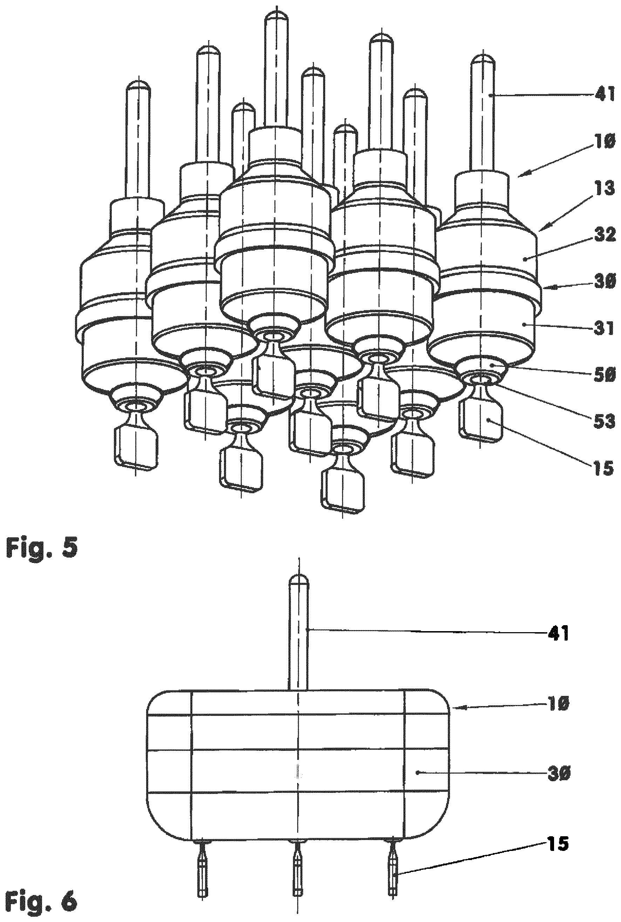

In der Figur 5 ist ein Injektionssystem (10) dargestellt, bei dem die z.B. neun Injektionseinheiten (13) gitterförmig angeordnet sind. Die Auslösevorrichtung und der Systemträger sind in der Figur 5 nicht dargestellt. Die einzelnen Injektionseinheiten (13) des Arrays sind beispielsweise identisch aufgebaut. Die Anzahl der Einheiten (13) im Array ist nur durch die Geometrie der Hautoberfläche des zu behandelnden Körpers begrenzt. Jede Austrittsöffung (53) einer Einheit (13) hat von den Austrittsöffnungen (53) zweier benachbarter Einheiten (13) jeweils den gleichen Abstand. Anstatt in der dargestellten Rasteranordnung können die Injektionseinheiten (13) des Arrays eine Dreiecks-, Rechteck-, Polygon- oder Ringfläche überdecken. Sie können auch entlang eines regelmäßigen oder unregelmäßigen, offenen oder geschlossenen Linienzuges angeordnet sein. Diese Injektionseinheiten (13) werden z.B. gemeinsam mittel einer Auslösevorrichtung betätigt. Einzelne Injektionseinheiten (13) können aber auch von der Auslösevorrichtung separat oder getrennt angesteuert sein.

Die Darstellung der Figur 5 zeigt das Injektionssystem (10) kurz vor dem Einsatz. Die Sterilverschlüsse (15) sind bereits von den Austrittsdüsen (53) abgezogen.

Die Figuren 6 - 8 zeigen ein Injektionssystem (10), bei dem die einzelnen Injektionseinheiten (13) in einem gemeinsamen Gehäuse (30) angeordnet sind. Jede Injektionseinheit (13) umfasst in diesem Ausführungsbeispiel einen Medikamentenbehälter (50) mit einer Austrittsdüse (53) und eine Treibladung (72; 73). Die einzelne Austrittsdüse (53) ist mittels eines Sterilverschlusses (15) verschlossen.

Das Gehäuse (30) umfasst ein verstärktes erstes (31) und ein zweites Gehäuseteil (32). Im Ausführungsbeispiel sind diese miteinander verklebt. In einem Gehäuseansatz (41) ist eine Zündladung (71) angeordnet. Über einen Durchbruch (44) des Deckenabschnitts (45) kommuniziert die Zündladung (71) mit dem Innenraum (46) des Gehäuses (30). Weitere Durchbrüche (47) sind in den Trennstegen (48) des Gehäuses (30) zwischen den einzelnen Injektionseinheiten (13) angeordnet, so dass alle Innenräume (46) mit den Treibladungen (72; 73) miteinander verbuden sind. In dem dargestellten Ausführungsbeispiel sind die Treibladungen (73) von der Zündladung (71) weiter entfernt als die Treibladungen (72). Im dargestellten Ausführungsbeispiel erfolgt die Verbindung der Zündladung (71) zu den einzelnen Treibladungen (72, 73) durch den Deckendurchbruch (44) und gegebenfalls durch einen oder zwei Wanddurchbrüche (47) hindurch.

Alle Medikamentenbehälter (50), ihre Austrittsöffnungen (53), die Schutzmembranen (60) und die jedem einzelnen Medikamentenbehälter (50) zugeordnete Treibladung (72; 73) sind so aufgebaut, wie im Zusammenhang mit dem in den Figuren 1 und 2 dargestellten Ausführungsbeispiel beschrieben.

Nach dem Aufsetzen z.B. auf die Haut kann das Injektionssystem (10) mittels einer hier nicht dargestellten Auslösevorrichtung betätigt werden. Diese Auslösevorrichtung kann so aufgebaut sein, wie im Zusammenhang mit dem ersten Ausführungsbeispiel beschrieben.

Durch das Betätigen der Auslösevorrichtung wird die einzige, z.B. zentral angeordnete Zündladung (71) dieses Injektionssystems (10) gezündet. Die heißen Abgase der Zündladung (71) wirken über die Durchbrüche (44, 47) auf die Treibladungen (72; 73). Hierbei kann beispielsweise über die Querschnitte der Durchbrüche (47) und/oder über die Abstände der einzelnen Einheiten (13) zur Zündladung (71) eine Kaskadierung der Treibladungen (72; 73) gesteuert werden. Damit führt beispielsweise zu einer sequentiellen Belastung der Haut. Bei Austrittsdüsen (53), die mit geringem Abstand zueinander angeordnet sind, wird außerdem eine gegenseitige Beeinflussung der Schusskanäle verhindert. Werden beispielsweise zehn Injektionseinheiten (13) kaskadiert nacheinander ausgelöst, ist die gesamte Injektionszeit für ein Volumen von 0,5 Milliliter kleiner als 1,5 Sekunden. Es ist aber auch eine gleichzeitige Zündung der Treibladungen (72; 73) denkbar. Hiermit wird ein großer Volumenstrom des Medikaments (5) in die Haut eingebracht. Die Entleerung der einzelnen Medikamentenkammern (50) erfolgt, wie im Zusammenhang mit dem ersten Ausführungsbeispiel beschrieben.

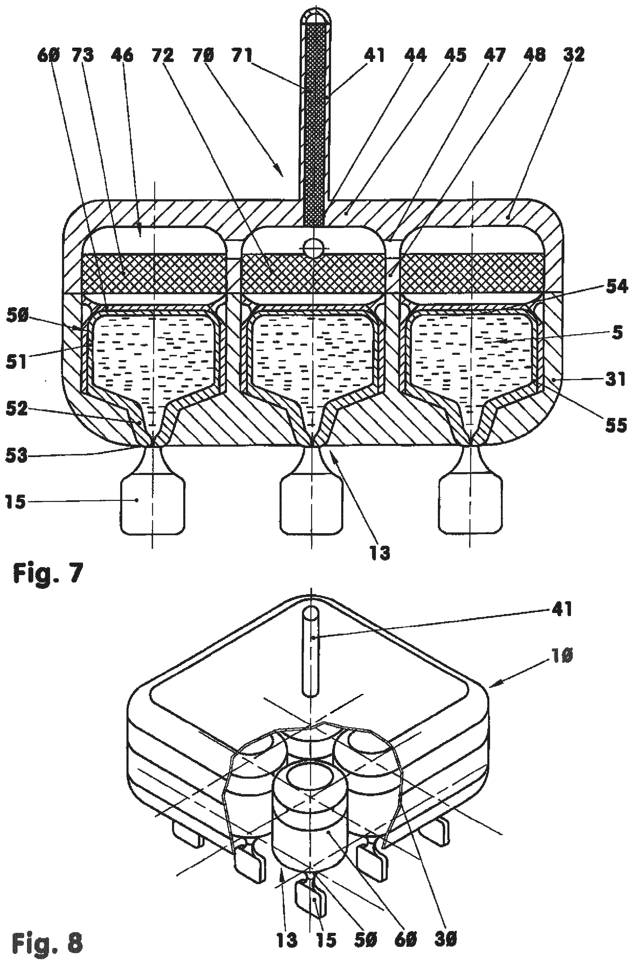

Die Figur 8 zeigt eine dimetrische Ansicht des in den Figuren 6 und 7 dargestellten Einmal-Injektionssystems (10). Dieses Injektionssystem (10) umfasst neun Injektionseinheiten (13). Analog zum Ausführungsbeispiel der Figuren 1 und 2 können auch diese Einheiten (13) linienförmig, ringförmig oder flächenförmig angeordnet sein. Das Injektionssystem (10) kann zwei oder mehr Einheiten (13) umfassen. Die Sterilverschlüsse (15) können einzeln sein oder miteinander verbunden sein.

Das Injektionssystem (10) kann statt des beschriebenen pyrotechnischen Antriebs auch Gaskartuschen oder Federn aufweisen. Auch bei einem derartigen Injektionssystem erfolgt das Auslösen mittels einer gemeinsamen Auslösevorrichtung,

Selbstverständlich ist es auch denkbar, die verschiedenen genannten Ausführungsformen miteinander zu kombinieren.

### Bezugszeichenliste:

- 5: Medikamentenlösung

- 10: Injektionssystem
- 11: Auslösevorrichtung
- 12: Taster
- 13: Einheit, Injektionseinheit

- 15: Sterilverschluss

- 20: Systemträger, Gehäuseträger
- 21: Abstützgestell
- 22: Trägerplatte
- 23: Abhebesicherung
- 24: Schrauben
- 25: Federelemente, Druckfedern
- 26: Rastverbindung
- 27: Aufnahmedurchbruch

- 30: Gehäuse
- 31: erster Gehäuseteil
- 32: zweiter Gehäuseteil
- 33: Ringnocken
- 34: Ringnut
- 35: Haltering
- 36: zylindrischer Abschnitt
- 37: konischer Abschnitt
- 38: zentraler Durchbruch
- 39: Innenwandung

- 41: Gehäuseansatz, Zündröhrchen
- 42: Ringbund
- 43: Raststifte
- 44: Durchbruch, Deckendurchbruch
- 45: Deckenabschnitt
- 46: Innenraum von (30)
- 47: Durchbrüche, Wanddurchbrüche
- 48: Trennstege

- 50: Medikamentenbehälter
- 51: Wandung
- 52: Austrittsabschnitt
- 53: Austrittsöffnung, Düse
- 54: Oberseite
- 55: Außenwandung
- 56: Kolben

- 60: Schutzmembran
- 61: Dichtlippe

- 70: Antriebsvorrichtung
- 71: Zündladung, Antriebselement von (70)
- 72: Treibladung, Antriebselement von (70)
- 73: Treibladung, Antriebselement von (70)

- 81: Leitung
- 82: Leitung

## Patentansprüche

1. Injektionssystem (10) mit einer mittels einer Auslösevorrichtung (11) auslösbaren Antriebsvorrichtung (70) und mit einem, mindestens eine Austrittsöffnung (53) aufweisenden Medikamentenbehälter (50) mit komprimierbarem Volumen, wobei die Antriebsvorrichtung (70) eine Zündladung (71) umfasst, wobei
- das Injektionssystem mindestens einen weiteren komprimierbaren Medikamentenbehälter (50) und eine weitere auslösbare Antriebsvorrichtung (70) aufweist,
- wobei jeweils eine auslösbare Antriebsvorrichtung (70) und ein mindestens eine Austrittsöffnung (53) aufweisender Medikamentenbehälter (50) mit einem komprimierbaren Volumen in einem gemeinsamen Gehäuse (30) angeordnet sind und wobei alle Antriebsvorrichtungen (70) mittels der gemeinsamen Auslösevorrichtung (11) betätigbar sind, **dadurch gekennzeichnet,**
**dass** die Gehäuse (30) in einem Systemträger (20) federnd gelagert sind.

2. Injektionssystem (10) nach Anspruch 1,
**dadurch gekennzeichnet,**
**dass** jedem Medikamentenbehälter (50) zumindest ein Antriebselement (72, 73) der Antriebsvorrichtung (70) und eine Austrittsöffnung (53) zugeordnet sind.

3. Injektionssystem (10) nach Anspruch 1,
**dadurch gekennzeichnet,**
**dass** die einzelne Austrittsöffnung (53) eine Austrittsdüse ist.

4. Injektionssystem (10) nach Anspruch 1,
**dadurch gekennzeichnet,**
**dass** die Wandung (51) mindestens eines Medikamentenbehälters (50) kompressibel ist.

5. Injektionssystem (10) nach Anspruch 1,
**dadurch gekennzeichnet,**
**dass** die Wandung (51) mindestens eines Medikamentenbehälters (50) starr ist und dieser Medikamentenbehälter (50) einen verschiebbaren Kolben (56) aufweist.

6. Injektionssystem (10) nach Anspruch 1,
**dadurch gekennzeichnet,**
**dass** jede Austrittsöffnung (53) zu mindestens zwei benachbarten Austrittsöffnungen (53) den gleichen Abstand hat.

7. Injektionssystem (10) nach Anspruch 1,
**dadurch gekennzeichnet,**
**dass** jedem Medikamentenbehälter (50) eine pyrotechnische Treibladung (72, 73) zugeordnet ist.

8. Injektionssystem (10) nach Anspruch 7,
**dadurch gekennzeichnet,**
**dass** der Abstand der Zündladung (71) zu mindestens zwei Treibladungen (72, 73) unterschiedlich groß ist.

9. Injektionssystem (10) nach Anspruch 7,
**dadurch gekennzeichnet,**
**dass** der Massenstrom zu mindestens zwei Treibladungen (72, 73) unterschiedlich groß ist.

10. Injektionssystem (10) nach Anspruch 1,
**dadurch gekennzeichnet,**
**dass** mindestens zwei Treibladungen (72, 73) unterschiedliche Verzögerungskonstanten aufweisen.

11. Injektionssystem (10) nach Anspruch 7,
**dadurch gekennzeichnet,**
**dass** sie mindestens zwei Injektionseinheiten (13) umfasst, die jeweils einen Medikamentenbehälter (50) und eine Treibladung (72, 73) aufweisen.

## Claims

1. Injection system (10) having a drive device (70) which is triggerable by means of a triggering device (11) and having a medicament container (50) which has at least one outlet opening (53) and a compressible volume, wherein the drive device (70) comprises a priming charge (71),
- wherein the injection system has at least one further compressible medicament container (50) and a further triggerable drive device (70),
- wherein a triggerable drive device (70) and a medicament container (50) which has at least one outlet opening (53) and a compressible volume are in each case arranged in a common housing (30), and wherein all of the drive devices (70) are actuable by means of the common triggering device (11), **characterized in that** the housings (30) are mounted resiliently in a system carrier (20).

2. Injection system (10) according to Claim 1, **characterized in that** at least one drive element (72, 73) of the drive device (70) and an outlet opening (53) are assigned to each medicament container (50).

3. Injection system (10) according to Claim 1, **characterized in that** the individual outlet opening (53) is an outlet nozzle.

4. Injection system (10) according to Claim 1, **characterized in that** the wall (51) of at least one medicament container (50) is compressible.

5. Injection system (10) according to Claim 1, **characterized in that** the wall (51) of at least one medicament container (50) is rigid, and said medicament container (50) has a displaceable plunger (56).

6. Injection system (10) according to Claim 1, **characterized in that** each outlet opening (53) has the same spacing from at least two adjacent outlet openings (53).

7. Injection system (10) according to Claim 1, **characterized in that** a pyrotechnic propellant charge (72, 73) is assigned to each medicament container (50).

8. Injection system (10) according to Claim 7, **characterized in that** the spacing of the priming charge (71) from at least two propellant charges (72, 73) differs in size.

9. Injection system (10) according to Claim 7, **characterized in that** the mass flow to at least two propellant charges (72, 73) differs in size.

10. Injection system (10) according to Claim 1, **characterized in that** at least two propellant charges (72, 73) have different delay constants.

11. Injection system (10) according to Claim 7, **characterized in that** it comprises at least two injection units (13) which each have a medicament container (50) and a propellant charge (72, 73).

## Revendications

1. Système d'injection (10) comprenant un dispositif d'entraînement (70) pouvant être déclenché au moyen d'un dispositif de déclenchement (11) et un récipient de médicament (50) présentant au moins une ouverture de sortie (53) avec un volume compressible, le dispositif d'entraînement (70) comprenant une charge d'allumage (71),
- le système d'injection présentant au moins un récipient de médicament compressible supplémentaire (50) et un dispositif d'entraînement pouvant être déclenché supplémentaire (70),
- à chaque fois un dispositif d'entraînement pouvant être déclenché (70) et un récipient de médicament (50) avec un volume compressible présentant au moins une ouverture de sortie (53) étant disposés dans un boîtier commun (30) et tous les dispositifs d'entraînement (70) pouvant être actionnés au moyen du dispositif de déclenchement commun (11),
**caractérisé en ce que**
les boîtiers (30) sont supportés de manière élastique dans un support de système (20).

2. Système d'injection (10) selon la revendication 1, **caractérisé en ce**
**qu'**au moins un élément d'entraînement (72, 73) du dispositif d'entraînement (70) et une ouverture de sortie (53) sont associés à chaque récipient de médicament (50).

3. Système d'injection (10) selon la revendication 1, **caractérisé en ce que**
l'ouverture de sortie individuelle (53) est une buse de sortie.

4. Système d'injection (10) selon la revendication 1, **caractérisé en ce que**
la paroi (51) d'au moins un récipient de médicament (50) est compressible.

5. Système d'injection (10) selon la revendication 1, **caractérisé en ce que**
la paroi (51) d'au moins un récipient de médicament (50) est rigide et ce récipient de médicament (50) présente un piston déplaçable (56).

6. Système d'injection (10) selon la revendication 1, **caractérisé en ce que**
chaque ouverture de sortie (53) présente la même distance à au moins deux ouvertures de sortie adjacentes (53).

7. Système d'injection (10) selon la revendication 1, **caractérisé en ce**
**qu'**une charge propulsive pyrotechnique (72, 73) est associée à chaque récipient de médicament (50).

8. Système d'injection (10) selon la revendication 7, **caractérisé en ce que**
la distance de la charge d'allumage (71) à au moins deux charges propulsives (72, 73) est différente.

9. Système d'injection (10) selon la revendication 7, **caractérisé en ce que**
le débit massique vers au moins deux charges propulsives (72, 73) est différent.

10. Système d'injection (10) selon la revendication 1, **caractérisé en ce**
**qu'**au moins deux charges propulsives (72, 73) présentent des constantes de décélération différentes.

11. Système d'injection (10) selon la revendication 7, **caractérisé en ce**
**qu'**il comprend au moins deux unités d'injection (13) qui présentent chacune un récipient de médicament (50) et une charge propulsive (72, 73).
